(19) Europäisches Patentamt
European Patent Office
Office européen des brevets

(11) Publication number: **0 035 304**
**B2**

(12) **NEW EUROPEAN PATENT SPECIFICATION**

(45) Date of publication of the new patent specification: **15.03.89**

(51) Int. Cl.⁴: **C 07 C 69/96**, C 07 C 68/06, C 08 F218/00

(21) Application number: **81200206.1**

(22) Date of filing: **20.02.81**

# (54) Process for preparing bis or tris allyl carbonates and process for preparing polymers thereof.

(30) Priority: **05.03.80 IT 2035180**

(43) Date of publication of application: **09.09.81 Bulletin 81/36**

(45) Publication of the grant of the patent: **01.02.84 Bulletin 84/05**

(45) Mention of the opposition decision: **15.03.89 Bulletin 89/11**

(84) Designated contracting states: **AT BE CH DE FR GB LI LU NL SE**

(56) References cited:
**EP--A-- 0 072 325**
**DE--A-- 1 493 757**
**FR--A-- 2 278 719**
**GB--A-- 1 274 837**
**US--A-- 2 384 115**
**US--A-- 2 563 771**
**US--A-- 2 915 529**
**US--A-- 4 146 522**
**Polymer 4/4, page 515-524 (1963)**
**S.F.O.S. Monomere CAD', Detcher 1984**
**Akzo Chemie "Nouryset [R] 200"**
**Bull. 45A, PPG Industries "CR-39", 58**
**Bull. 45A, PPG Industries "CR 39", 64**
**Kirk-Othmer, "Encyclopedia of Chemical Technology", 3rd Ed., vol. 2, p. 111-113 (1978)**
**"Process Evaluation and Research Planning"-First Quarterly Report 1982/1983 Program October 1982, p.70,71,74, Chem. Systems, New York 10591**
**Ind. Eng. Chem., Vol. 47, p. 2447 (1955)**
**L.H. Peebles Jr. "Molecular Weight Distribution in Polymers", Interscience (1971), p. 190-196**
**Kirk-Othmer "Encyclopedia of Chemical Technology", 3rd Edition (1980), vol. 9, p. 291-310**
**S.F.O.S. letter of 20.09.85 "Allyldiglycol carbonate /XR 80"**
**The file contains technical information submitted after the application was filed and not included in this specification**

(73) Proprietor: **ANIC S.p.A.**
**Via M. Stabile, 216**
**I-90139 Palermo (IT)**

(72) Inventor: **Romano, Ugo**
**Via 25 Aprile, 10**
**I-20059 Vimercate (Milano) (IT)**
Inventor: **Iori, Giuseppe**
**Via Mattei, 48**
**I-20097 San Donato Milanese (Milano) (IT)**

(74) Representative: **Freiherr von Pechmann, Eckehart et al**
**Patentanwälte Wuesthoff- v. Pechmann-Behrens-Goetz Schweigerstrasse 2**
**D-8000 München 90 (DE)**

**EP 0 035 304 B2**

## Description

This invention relates to a process for preparing a mixture of monomeric and oligomeric bis or tris (allyl carbonates) useful as optical-glass substitutes due to the high factor of transparency they possess when in their polymerized form.

The invention also relates to a process for preparing polymers thereof according to claim 4.

For the preparation of bis- and tris(allyl carbonates), and for the preparation of allyl carbonates from polyhydric alcohols and poly-basic acids as the starting materials, the prior art offers a number of processes which, as will be seen shortly hereinafter, have a number of drawbacks which render these products of the prior art objectionably unsuitable for applications in which a high optical transparency is required, such as for the manufacture of substitutes for optical glasses, like lenses, condensers, prisms and like articles.

Exemplary of this prior art are the US Patent specifications 2 384 115, 2 370 565 and 2 592 058.

The feature which is common to all of these prior art disclosure is that, according to their teachings, allyl carbonates derived from polyhydric alcohols and polybasic acids were prepared by a classical reaction consisting of a transesterification, the reactants of which were, typically enough, a chloroformate and a polyhydric alcohol.

These prior art approaches are open to objection, in general, because chloroformates are, as is well known, irritating and toxic compounds and, above all, phosgene is used in their preparation and it is hardly necessary to recall that phosgene is a well known chemical warfare agent.

On the top of that, there is a fact which deserves special attention and is that the chlorine present in chloroformates has a detrimental effect on the transparency of the finished products so that expensive and cumbersome purification runs were required, as a rule, in order to obtain optically acceptable allyl carbonate products.

The same applies to commercial diethylene glycol bis (allyl carbonate) prepared by reaction of diethylene glycol bis (chloroformate) with allyl alcohol in the presence of NaOH according to Encyclopedia of Chemical Technology by Kirk Othmer, 3rd Edition, Vol. 2., p. 111/112 and known as CR 39, which is investigated in the Essilor Report (1977). According to this document CR39 contains varying amounts of oligomers and prepolymers. The varying composition of this product is a disadvantage for the user. According to FR-A 2 278 719 an α,ω-bis-(allyl carbonate) is prepared by first condensing diethylene glycol bis (chloroformate) with diethylene glycol and then reacting the obtained diol polycarbonate with allyl chloroformate. The desired colourless allyl polycarbonate is obtained in a yield of 65 %, calculated on the starting compound from step one.

US-A-2 563 771 discloses a process for the preparation of bis (allyl carbonates) of unsaturated diols by reacting the unsaturated diols with diallyl carbonate in the presence of an alkaline catalyst. The reaction products have to be taken up with benzene and then distilled and are used for polymerizing into resinous products, which may be used to prepare glass substitutes and for interlayers or adhesives in safety glass.

Another transesterification reaction between carbonate esters and diols is known from US-A-4 146 522 whereby, when starting from diethyl carbonate and 1.4-butandiol or 1.6-hexandiol, respectively, a mixture of the respective glycol bis(ethyl carbonate) and polyglycol diethyl carbonate is obtained which is used in a smoke suppressant composition.

The serious shortcomings of the prior art methods and products useful as optical glass substitutes can both safely and conveniently be offset by adopting the process according to the present invention for the preparation of a mixture of monomeric and oligomeric bis or tris (allyl carbonates) by a transesterification reaction, characterized in that diallyl carbonate is reacted with a special diol, or a triol as the case may be, the molar ratio of the diallyl carbonate to the diol, or the triol, concerned being comprised in the range from 2 : 1 to 20 : 1, at a temperature of from 50 °C to 150 °C and with a catalyst of alkaline nature being present in an amount of at least 0.1 ppm (part per million) relative to the diol, or triol, concerned, on a weight basis.

Preferable catalysts are sodium hydroxide, sodium carbonate, a sodium alcoholate, an organic base and a basic ion-exchange resin.

The process comprises carrying out the transesterification reaction under a pressure of between atmospheric pressure and 13,33 mbar (10 mmHg).

The diols and triols, hereinafter also named polyhydric alcohols, used in the process of this invention, are selected from the group consisting of ethylene glycol, propylene glycol, diethylene glycol, triethylene glycol, tetraethylene glycol, butanediol, hexanediol and glycerol. The reaction can thus be carried out using commercially available products as the starting substance without further purification.

The final products are absolutely colourless and free from those impurities which, as stated, lead to the disadvantages of the products obtained according to the known art.

With the present method of synthesis, a mixture of the monomeric ester with oligomers constituted by chains of allyl terminated alcohol polycabonate is obtained, according to the diallyl carbonate/polyhydric alcohol molar ratio. The structure of the monomer and oligomers in the case of glycol is as follows :

$$CH_2{=}CH{-}CH_2{-}O\underset{\underset{O}{\|}}{C}O(RO\underset{\underset{O}{\|}}{C}O)_nCH_2{-}CH{=}CH_2$$

where n lies between 1 and 10, and R represents a hydrocarbon radical, which can be interrupted by oxygen atoms and is then derived from diethylene glycol, triethylene glycol or tetraethylene glycol.

If the diallyl carbonate/glycol ratio is greater than or equal to 10, the final product is formed by the bis (allyl carbonate) of the glycol with a quantity of less than or equal to 10 % of oligomers mainly represented by dimers (n = 2). If the ratio is less than 10, the oligomer percentage rises until, for an approximately stoichiometric ratio, it reaches a value close to 70 % of the reaction product.

The density and viscosity of the product increase in relation to the increase in oligomer concentration.

By way of example, some characteristics of the product obtained from diallyl carbonate (DAC) and diethylene glycol (DEG) for various molar ratios are as follows :

| Mol ratio. DAC/DEG | % monomer | % oligomers | Density | Viscos. $mm^2/s$ (25°C) |
|---|---|---|---|---|
| 12 | 94 | 6 | 1.148 | 12 |
| 8 | 81 | 19 | 1.151 | 17 |
| 5 | 60 | 40 | — | 19 |
| 3.5 | 52 | 48 | — | 32 |
| 2.5 | 40 | 60 | — | 64 |

The Saybolt colour is constantly greater than + 30, and the UV-visible absorbency is a follows :

| nm | 300 | 350 | 400 | 450 | 600 | 700 |
|---|---|---|---|---|---|---|
| Absorbency | 0.2 | 0.04 | 0 | 0 | 0 | 0 |

The only volatile impurities (b.p. < 150° at 6.67 mbar) which can be present are traces of diallyl carbonate (< 1 %).

According to one embodiment of the process of the invention, the reaction between diallyl carbonate and the polyhydric alcohol is carried out in a vessel fitted with a stirrer and distillation column for removing the allyl alcohol released by the transesterification reaction.

The carbonate and alcohol are thus fed in the required molar ratio, and the environment is deaerated before introducing the catalyst. With diethylene glycol, for example, the catalyst is added in dispersed solid form or in preferably alcoholic solution to the extent of 0.5 to 10 ppm by weight of Na with respect to the diethylene glycol used.

Heating is then started under a residual pressure of 199.95-266,6 mbar taking particular care to have no infiltration of air. The allyl alcohol, which is released to the extent of 2 moles per mole of fed I glycol, rapidly distils over, and the reaction is terminated in about 1 hour. The residual vacuum is then gradually increased to remove the excess of diallyl carbonate. The diallyl carbonate which remains in the product is a function of the degree of vacuum at which this removal is carried out. In particular, if operating at 13,33 mbar the diallyl carbonate remaining in the product is less than 1 %.

The residual product, after filtering and possibly washing with water and drying, is perfectly clear, colourless and suitable for the application of which it is intended. The esters thus obtained can for example be directly used in radical polymerisation reactions in bulk, to give products of high technological value.

In the particular case of polymerisation, this is carried out in the presence of initiators or free radicals of peroxide or peroxycarbonate type in a percentage variable between 1 and 12 % with respect to the monomer, at a temperature of between 30 and 120 °C for a time variable from a few hours to several hours.

The moulds of the required shape, which are usually of carefully machined glass or steel fitted with an elastic gasket in order to follow the volume contraction of the product under polymerisation, are completely filled with the monomer to which the filtered and deaerated catalyst is added.

They are then placed in an air or water oven and left there to polymerise for a time and temperature cycle which vary with the dimensions of the mould, the thickness, the type and percentage of catalyst.

Generally, an increasing temperature of between 40 and 100 °C is maintained for a time varying from a few hours to several tens of hours.

At the end of the determined cycle, the product, which is now at the termination of cross-linkage, can be released from the mould and subjected to final hardening by heat treatment in air ovens at a temperature of about 90-110 °C for a time of between 1 hour and a few hours. The products thus treated attain a very high and constant quality standard.

The measurement of the chemical and physical characteristics of the various products demonstrates a complete and properly conducted polymerisation cycle.

Using the described polymerisation method, test pieces uniform in terms of shape and dimensions were obtained, and these were then subjected to measurements of optical-mechanical properties significant for the main use for which these polymers are intended, i. e. as glass substitutes.

The polymerisation for example of the various samples of carbonic allyl esters of diethylene glycol always followed the scheduled temperature and duration cycles, and never gave rise to any difficulties during cross-linkage, or on opening the moulds, such as breakages or separation difficulties. The transmission values for light from 350 to 700 nm are constantly above 89 %.

The Rockwell hardness measured on test pieces deriving from distilled ester is maintained at around values of M100, while falling to values of M85 for test pieces originating from ester containing 70 % of monomer and 30 % of oligomer carbonates, and reducing to values of M50 for test pieces originating from ester in which the oligomer carbonates represent 60 % of the product.

The resistance to scratching is high for all samples, and this appears substantially independent of the percentage of oligomers in the initial allyl carbonate ester.

However, the impact resistance increases considerably with this percentage. The bending modulus is also constant within certain limits, and is reduced for test pieces originating form esters containing 60 % of oligomers.

## Example 1

12 moles of diallyl carbonate and 1 mole of diethylene glycol are mixed at ambient temperature under an inert atmosphere in a 3 neck flask fitted with a thermometer, stirrer and distillation column. When mixing is complete, 0.05 millimoles of powdered NaOH are added, and heating is commenced at a residual pressure of 199.95 mbar.

After the two moles of released allyl alcohol have been withdrawn as overheads, the residual pressure is lowered to 2.67 mbar in order to remove the excess diallyl carbonate. A perfectly colourless product (Saybolt colour > + 30) is obtained on the bottom, and is composed of

| | |
|---|---|
| Diallyl carbonate | 0.5 % by weight |
| Diethylene glycol bis (allyl carbonate) | 89.85 % by weight |
| Oligomers | 9,65 % by weight |

This product is washed with water until neutral, dried and filtered. The yield with respect to the fed diethylene glycol is total.

## Example 2

The reaction is carried out as in example 1, with the difference that 0.005 millimoles of sodium methylate are used as catalyst. The sodium methylate was introduced as a 30 % methanol solution, again under an inert atmosphere.

When the reaction was finished, the colourless bottom liquid was washed, dried and filtered.

The yield is total with respect to the fed diethylene glycol.

The composition of the product is :

| | |
|---|---|
| Diallyl carbonate | 0.5 |
| Diethylene glycol bis (allyl carbonate) | 91.5 |
| Oligomers | 8.0 |

## Example 3

The reaction is carried out as in example 2, the only difference being that the catalyst was introduced as a solid, and after removing the excess of diallyl carbonate the perfectly colourless bottom product is only filtered. Yield and composition as in example 2.

## Example 4

The reaction is carried out as in example 2, except that the diallyl carbonate : diethylene glycol molar ratio was 10 : 1. The perfectly colourless bottom product is washed, dried, filtered and analysed. The yield

with respect to the fed diethylene glycol is total. The composition is as follows :

| | |
|---|---|
| Diallyl carbonate | 0.5 |
| Diethylene glycol bis (allyl carbonate) | 85.3 |
| Oligomers | 14.2 |

Viscosity at 25 °C 15.15 $mm^2/s$

Examples 5-8

The reaction described in example 4 is carried out with the following diallyl carbonate/diethylene glycol ratios

| Example | Diallyl carbonate/ diethylene glycol ratio | Composition | | Viscosity $mm^2/s$ at 25°C |
|---|---|---|---|---|
| | | Monomer | Oligomers | |
| 5 | 8:1 | 81 | 19 | 17 |
| 6 | 5:1 | 60 | 40 | 19 |
| 7 | 3.5:1 | 52 | 48 | 32 |
| 8 | 2.5:1 | 40 | 60 | 64 |

Example 9

The reaction is carried out as in example 2, the only difference being that 0.01 millimoles of metal sodium per mole of diethylene glycol are used as catalyst.

After the excess diallyl carbonate has been removed, a slightly yellow bottom product of identical composition to the product of test 2 remains.

Purification is carried out by distilling at a residual pressure of 2.67 mbar and a temperature of 160 °C.

Perfectly colourless diethylene glycol bis (allyl carbonate) is obtained, with a yield of 80 % with respect to the fed diethylene glycol.

A very viscous yellow product remains in the bottom, consisting mainly of allyl terminated oligomer carbonates.

Examples 10-13

The peroxide-based initiator was added to the esters obtained in the tests of the preceding examples, which were then polymerised in glass moulds fitted with flexible gaskets in air ovens or water baths at a temperature increasing between 40 and 90 °C for a time of between 2 and 24 hours according to the type and concentration of initiator. Test pieces were obtained, of which both the optical properties and physicomechanical properties were measured.

(See table page 6)

| Example No. | bis(allyl carbonate) | Optical properties | | | Physico-mechanical properties | | | |
|---|---|---|---|---|---|---|---|---|
| | | Visible Transmission (%) (1) | Rockwell Hardness M (2) | Izod Impact Notched J/m (3) | Izod Impact Unnotched kJ/m² (4) | Elastic Modulus MPa (5) | Scratch Resistance (6) | Taber Abrasion % Haze (7) |
| 10 | Ex.9 | 90.5 | M 97 | 28 | 170 | 2200 | 32 | 35 |
| 11 | Ex. 2 | 90.0 | M 90 | 35 | 230 | 2100 | 32 | 33.5 |
| 12 | Ex. 5 | 91.0 | M 83 | 50 | 230 | 2100 | 29 | — |
| 13 | Ex. 8 | 90.8 | M 50 | 40 | 320 | 700 | 24 | — |
| Polycarbonate from | | | | | | | | |
| bisphenol A | — | | — | — | — | — | 6 | |
| Polystyrene | — | | — | — | — | — | 6 | |

(1) Thickness, 2,7 mm at 400-700 nm
(2) ASTM D 785
(3) ASTM D 256
(4) ASTM D 256 MODIF
(5) ASTM D 790
(6) INTERNAL METHOD
(7) CS10 ABRASIVE WHEEL, load 0.5 kg.

**Claims**

1. Process for the preparation of a mixture of monomeric and oligomeric bis- or tris (allyl carbonates) by a transesterification reaction, characterized in that diallyl carbonate is reacted with a diol or a triol selected from the group consisting of ethylene glycol, propylene glycol, diethylene glycol, triethylene glycol, tetraethylene glycol, butanediol, hexanediol and glycerol, in a molar ratio of the diallyl carbonate to the diol or the triol comprised in the range from 2 : 1 to 20 : 1, at a temperature of from 50 to 150 °C and in the presence of at least 0.1 ppm (part per million) of an alkaline catalyst, calculated on the weight of the diol or triol.

2. A process according to claim 1, characterized in that said alkaline catalyst is selected from the group consisting of sodium hydroxide, sodium carbonate, a sodium alcoholate, an organic base and a basic ion-exchange resin.

3. A process according to claim 1 or 2 characterized in that said transesterification reaction is carried out under a pressure of between atmospheric pressure and 13.33 mbar (10 mm Hg).

4. A process for preparing polymers useful as optical-glass substitutes, characterized in that the mixture of colourless monomeric and oligomeric bis (allyl carbonates) obtained in the process of claims 1 to 3 is directly used as starting material in a polymerization carried out at a temperature of from 30 to 120 °C in the presence of 1 to 12 % by weight with respect to the starting material of a radical initiator selected from the group consisting of peroxides and peroxycarbonates.

**Patentansprüche**

1. Verfahren zur Herstellung eines Gemischs von monomeren und oligomeren Bis- oder Tris (allylcarbonaten) durch eine Umesterungsreaktion, dadurch gekennzeichnet, daß man das Diallylcarbonat mit einem Diol oder einem Triol aus der Gruppe mit Ethylenglykol, Propylenglykol, Diethylenglykol, Triethylenglykol, Tetraethylenglykol, Butandiol, Hexandiol und Glyzerin bei einem molaren Verhältnis zwischen dem Diallylcarbonat und dem Diol oder Triol im Bereich von 2 : 1 bis 20 : 1 bei einer Temperatur zwischen 50 °C und 150 °C und in Gegenwart eines alkalischen Katalysators in einer Menge von wenigstens 0,1 ppm (Teile je Million), bezogen auf das Gewicht des Diols oder Triols, umsetzt.

2. Verfahren nach Anspruch 1, dadurch gekennzeichnet, daß der alkalische Katalysator aus der Gruppe mit Natriumhydroxid, Natriumcarbonat, einem Natriumalkoholat, einer organischen Base und einem basischen Ionenaustauschharz gewählt wird.

3. Verfahren nach Anspruch 1 oder 2, dadurch gekennzeichnet, daß die Umesterungsreaktion bei einem Druck zwischen dem Atmosphärendruck und 13,33 mbar (10 mm HG) durchgeführt wird.

4. Verfahren zum Herstellen von als Ersatz für optisches Glas geeigneten Polymeren, dadurch gekennzeichnet, daß das in dem Verfahren gemäß Anspruch 1 bis 3 erhaltene Gemisch von farblosen monomeren und oligomeren Bis (allylcarbonaten) direkt als Ausgangsmaterial in einer Polymerisation verwendet wird, die bei einer Temperatur zwischen 30 °C und 120 °C in Gegenwart eines Radikalinitiators aus der Gruppe mit Peroxiden und Peroxycarbonaten in einer Menge von 1 bis 12 Gew.-%, bezogen auf das Ausgangsmaterial, durchgeführt wird.

**Revendications**

1. Procédé de préparation d'un mélange de bis- ou tris- (carbonates d'allyle) monomères et oligomères par une réaction de transestérification, caractérisé en ce que l'on fait réagir du carbonate de diallyle avec un diol ou un triol choisi dans le groupe comprenant l'éthylène glycol, le propylène glycol, le diéthylène glycol, le triéthylène glycol, le tétraéthylène glycol, le butanediol, l'hexanediol et le glycérol, avec un rapport molaire entre le carbonate de diallyle et le diol ou le triol compris entre 2 : 1 et 20 : 1, à une température allant de 50 °C à 150 °C et en présence d'un catalyseur de nature alcaline dans une quantité d'au moins 0,1 ppm (parties par million), calculée sur le poids du diol ou du triol.

2. Procédé selon la revendication 1, caractérisé en ce que ledit catalyseur de nature alcaline est choisi dans le groupe comprenant l'hydroxyde de sodium, le carbonate de sodium, un alcoolate de sodium, une base organique et une résine basique échangeuse d'ions.

3. Procédé selon la revendication 1 ou 2, caractérisé en ce que ladite réaction de transestérification est exécutée à une pression comprise entre la pression atmosphérique et 13,33 mbar (10 mm Hg).

4. Procédé de préparation de polymères pouvant se substituer au verre optique, caractérisé en ce que le mélange de bis (carbonates d'allyle) monomères et oligomères incolores, obtenu dans le procédé selon les revendications 1 à 3, est directement employé comme matériau de base dans une polymérisation exécutée à une température allant de 30 °C à 120 °C en présence d'un initiateur de radicaux choisi dans le groupe comprenant des peroxydes et des peroxycarbonates, la quantité dudit initiateur de radicaux étant comprise entre 1 % et 12 % en poids par rapport au matériau de base.